# EUROPEAN PATENT SPECIFICATION

(11) **EP 3 090 726 B1**
(45) Date of publication and mention of the grant of the patent: **29.05.2019**
(21) Application number: 15201522.8
(22) Date of filing: 21.12.2015
(51) Int. Cl.: A61K 8/33, A61K 8/46, A61K 8/49, A61Q 19/00

(54) **COSMETIC COMPOSITION COMPRISING A COMBINATION OF ACTIVE SUBSTANCES**
KOSMETISCHE ZUSAMMENSETZUNG MIT EINER KOMBINATION VON WIRKSTOFFEN
COMPOSITION COSMÉTIQUE COMPRENANT UNE COMBINAISON DE SUBSTANCES ACTIVES

(43) Date of publication of application: 09.11.2016
(73) Proprietor: Thiocyn GmbH, 60313 Frankfurt (DE)
(72) Inventor: STROTHMANN, Rene, 49186 Bad Iburg (DE)
(74) Representative: f & e patent

(56) References cited:
- EP-A1- 1 287 814
- WO-A1-98/40047
- WO-A2-98/57614

## Description

The present invention refers to a cosmetic composition, comprising Allantoin and Heliotropine, and ionically bound and/or free thiocyanate ions or derivatives thereof, a combination of Allantoin and/ Heliotropine and ionically bound and/or free thiocyanate ions or derivatives thereof for use in the treatment of stressed skin, e.g. by dermatitis, neurodermitis, psoriasis, eczema, allergic reaction, heat, cold, sunburn and/or dryness and the use of ionically bound and/or free thiocyanate ions or derivatives thereof in cosmetic compositions for improved effectiveness of incorporation of Allantoin and Heliotropine in said cosmetic compositions.

Cosmetic compositions are widely available and are used in general for cleaning, refreshing and shaping parts of the human body (for example the hair or the skin) or for changing or decorating the appearance of the skin and the hair.

But the permanent use of personal care products and cosmetic compositions on hair and skin like e.g. soaps, shampoos, shower gels, shower foams or sprays has a negative influence on the hair and skin appearance. Furthermore, the aggressions of the environment, including climate aggressions such as exposure to sunlight, temperature variations, pollution, stress, fatigue, and aging in general tend to affect the natural regeneration capacity and appearance of skin and/or hair. Besides these external stress factors that might be caused by e.g. sunburn, dryness or allergic reactions, the skin might be as well affected by variety of conditions like e.g. dermatitis, neurodermitis, psoriasis, or eczema. The result of any of these influences might be stressed skin with uncomfortable itching or irritation.

The present application aims at providing skin and hair caring cosmetic products for irritated or stressed skin in order to overcome the above described discomfort.

A variety of skin caring agents is known in the field and is used in a wide range of formulations and products on the market. For example the skin caring properties of urea or its derivatives is used in many formulations as the following effects are known for urea or its derivatives in amounts of at least 2 wt.-%: hydration of the stratum corneum, keratolytic properties, acceleration of penetration, inhibition of epidermal proliferation (basal cells), low antimicrobial activity, and buffering effect/regulation of the hydrolipid mantle (Wohlrab W. (1989), Bedeutung von Harnstoff in der externen Therapie, Hautarzt 40, Suppl. 9: 35-41). In modern dermatotherapy urea is successfully used as a single agent or as an additive in the healing phase of dermatosis.

In DE 43 41 001 A1 an urea content of 0.01 to 30 wt.-%, preferably from 0.1 to 10 wt .-%, is disclosed for topical compositions, which are used as prophylaxis and/or in the treatment of dry and scaly skin in cosmetic preparations and in the treatment of skin diseases like e.g. neurodermitis or psoriasis.

Another agent used in skin caring products is thiocyanate. For example the accelerated healing of skin wounds is described in EP 1 287 814 EP, while DE 41 34 888 A describes the accelerated healing of UV-induced erythema.

US 3,384,548 discloses the combined use of urea and thiocyanate as a swelling agent for the depilation of hair. Said application describes the use of 2 to 7.5 wt .-% urea and 4 wt .-% thiocyanate.

WO 98/57614 describes the combined use of thiocyanate ions and urea in a ratio of 1:10 to 1:50. Said combination and ratio is described as fostering the defense ability of healthy or stressed skin.

EP 1 287 814 A1 describes an opposite ratio between thiocyanate ions and urea in order to foster the defense capacity of the skin against chemical and physical irritations. Said application describes a weight ratio of thiocyanate ions to urea and/or urea derivatives in the range from 10:1 to 250:1. Urea derivatives used in this connection include amongst others carbamide and Allantoin.

It was an objective of the present invention to provide cosmetic compositions providing a stable skin caring performance, in particular for irritated or stressed skin.
For this purpose, several ingredients were focussed to be incorporated into a skin caring composition. Two of the preferred ingredients to provide skin caring properties to the cosmetic composition were Allantoin and/or Heliotropine.
However, during the preparation and testing of skin caring and hair caring products including at least one of theses two, it was found that it was difficult to distribute Allantoin and Heliotropine stably uniformly within such compositions. Especially a combination of Allantoin and Heliotropine appeared quite difficult to distribute within the cosmetic composition in a way that a permanently caring effect of the cosmetic composition was ensured.

Further ingredients were also tested for use as skin caring agent in a suitable composition, e.g thiocyanate salts. Several combinations of the ingredients of interest were considered under the focus of optimal caring properties for the cosmetic composition. It was suprisingly found that an accessory addition of thiocyanate into a cosmetic composition including Allantoin and/or Heliotropine improves the effectiveness of incorporation and distribution of said Allantoin and/or Heliotropine within the cosmetic composition. Further said distribution can be maintained during storage. In particular if both components were present the uniform distribution of Allantoin and Heliotropine in the cosmetic composition was clearly increased by the addition of thiocyanate to said cosmetic compositions, comprising Allantoin and Heliotropine.

Hence, the above-mentioned object is met, according to the invention, by a cosmetic composition, comprising
a) Allantoin and Heliotropine, and
b) ionically bound and/or free thiocyanate ions or derivatives thereof.

In terms of the present invention, "cosmetic composition" refers to any composition that is non-medical and non-pharmaceutical, hence, to a composition that is not intended for use in the treatment of a disease. The cosmetic compositions according to the present invention are therefore referring in particular to the treatment of the skin in terms of care without intending to treat a disease condition aiming at the healing of said disease. Hence, "cosmetic composition" refers to a composition that has an effect on the appearance and comfort of skin and/or hair without being used as a pharmaceutical or medicament.

Allantoin as an active ingredient in cosmetics is known for several beneficial effects, including a moisturizing and keratolytic effect, increasing the water content of the extracellular matrix and enhancing the desquamation of upper layers of dead skin cells, increasing the smoothness of the skin, promoting cell proliferation and wound healing. Therefore, Allantoin is commonly used in cosmetic lotions and creams, clarifying lotions, sun care products, lipsticks, shampoos and oral hygiene products. Furthermore, pharmaceutical formulations benefit from its inflammatory-modulating properties and effects on fibroblast proliferation and synthesis of the extracellular matrix.

Allantoin has the following chemical structure with the formula C₄H₆N₄O₃:

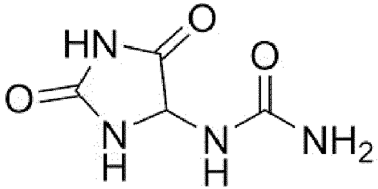

And is also known as (2,5-Dioxo-4-imidazolidinyl) urea (IUPAC name), Glyoxyldiureide and 5-Ureidohydantoin.

A further ingredient commonly used in such caring products due to its skin caring properties is Heliotropine. Heliotropine naturally occurs in various plants, like e.g. vanilla, violet flowers and black pepper, and is often found in fragrances and flavors. Therefore, besides its skin caring properties, Heliotropine is used in perfume, soap and detergent formulations due its pleasant scent.

Heliotropine is also known as Piperonal and has the chemical formula C₈H₆O₃ with the following structure:

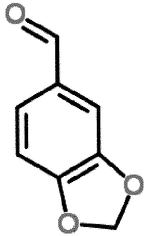

Further names of Heliotropine are Piperonyl aldehyde, Protocatechuic aledehyd methylene ether, 3,4-methylenedioxybenzaldehyde or 1,3-Benzodioxole-5-carbaldehyde as IUPAC name.

It was the intention of the inventors to include both of these compounds (Allantoin and Heliotropine) in the skin caring agent of the invention.

The uniform distribution of Allantoin and Heliotropine in the cosmetic composition was found to cause problems during the preparation and during storage of such caring products. However, a non-uniform distribution is not desirable since in this case the skin-caring effect might not be generated permanently during use. Rather, consumers expect from skin and hair caring products a high quality of skin caring on a constant level, even after longer storage times.

It appeared that during preparation, and further during storage of the cosmetic composition the Allantonin and the Heliotropin are not uniformly dispersed throughout the whole cosmetic composition, or don't remain uniformly distributed.

Without wishing to be bound to the theory it is assumed that it may happen during preparation and/or during storage of the cosmetic composition that hydrogen bridges are formed between the single molecules of Allantoin and / or Heliotropin, which is particularly present when both types of molecules are present in the composition. By forming such hydrogen bridges the molecules form larges complexes instead of a uniform distribution of the single molecules within the composition. In particular during storage of the composition (up to or during use) the components may form further hydrogen bridges.

It was found by the inventors that said problem of distribution can be overcome by the accessory addition of thiocyanate during the preparation of said cosmetic compositions. An improved effectiveness of incorporation and uniform distribution of Allantoin and Heliotropine within the cosmetic composition could be achieved and maintained during storage upon addition of thiocyanate. Said thiocyanate may be added as a suitable skin-acceptable salt.

Thiocyanate is also known as rhodanide or - under the IUPAC nomenclature - as cyanosulfanide. The chemical formula of thiocyanate is SCN-. According to the present invention the thiocyanate ions are used in form of alkali metal salts, ammonium salts or derivatives thereof, preferably the alkali metal salts of thiocyanates are selected from sodium thiocyanate, potassium thiocyanate and/or ammonium thiocyanate, more preferably the alkali metal salts of thiocyanates are selected from sodium thiocyanate and potassium thiocyanate.

The term "ionically bound" refers to the chemical bond that involves electrostatic attraction between oppositely charged ions. According to the present invention the thiocyanate ion SCN- as anion may be ionically bound to any monovalent cation, e.g. a metal cation M⁺ to form a complex of the general formula M⁺SCN⁻. According to the present invention the thiocyanate ions are preferably in form of alkali metal salts or derivatives thereof or ammonium salts or derivatives thereof or mixtures of alkali metal salts or derivatives thereof and ammonium salts or derivatives thereof, preferably the alkali metal salts of thiocyanates are selected from sodium thiocyanate, potassium thiocyanate and/or ammonium thiocyanate, more preferably the alkali metal salts of thiocyanates are selected from sodium thiocyanate and potassium thiocyanate. On the opposite "free thiocyanate ions" refer to unbound thiocyanate ions. In this connection, the term "derivatives thereof refers to thiocyanate ions bound to any possible alkali metal salt.

According to the invention, the cosmetic composition comprises Allantoin and Heliotropine and ionically bound and/or free thiocyanate ions or derivatives thereof.

The above mentioned ingredients may be incorporated in any cosmetic composition comprising further ingredients commonly used for the preparation of cosmetic compositions. Such cosmetic compositions can be represented by e.g. creams, lotions, emulsions, body milks, body sprays, unguents, ointments and pastes, but further as well detergent compositions like shower gels, hair shampoo or conditioner, or antiperspirants (deodorizing compositions), or other body care products. Further, decorative cosmetic products like make-up, rouge or tanning compositions may represent cosmetic compositions according to the invention, without being limited to these.

The cosmetic composition of the present invention may be applied to mammalian keratinous tissue, in particular to human skin. The cosmetic compositions may have various forms, for example solutions, suspensions, lotions, creams, gels, toners, sticks, pencils, sprays, aerosols, ointments, cleansing liquid washes and solid bars, shampoos and hair conditioners, pastes, foams, powders, mousses, shaving creams, wipes, strips, patches, wound dressing and adhesive bandages, hydrogels, film-forming products, facial and skin masks, decorative cosmetics (e.g. foundations, eye liners, eye shadows), and the like.

Thus, the cosmetic composition preferably further comprises a dermatologically acceptable carrier wherein the Allantonin / Heliotropin and thiocyanate are incorporated to enable the compounds (and optional other ingredients) to be delivered to the skin. The carrier may further comprise any suitable ingredients usually used in according compositions.

The carrier may contain one or more dermatologically acceptable, hydrophilic diluents. Hydrophilic diluents include water, organic hydrophilic diluents such as lower monovalent alcohols (e.g., C1-C4) and low molecular weight glycols and polyols, including propylene glycol, polyethylene glycol (e.g., molecular weight 200-600 g/mole), polypropylene glycol (e.g., molecular weight 425-2025 g/mole), glycerol, butylene glycol, 1,2,4-butanetriol, sorbitol esters, 1,2,6-hexanetriol, ethanol, isopropanol, sorbitol esters, butanediol, ether propanol, ethoxylated ethers, propoxylated ethers and combinations thereof. Carriers may also be in the form of an emulsion, such as oil-in-water emulsions, water- in-oil emulsions, and water-in- silicone emulsions. An emulsion may generally be classified as having a continuous aqueous phase (e.g., oil-in-water and water-in-oil-in- water) or a continuous oil phase (e.g., water- in-oil and oil-in- water- in-oil). The oil phase may comprise silicone oils, non-silicone oils such as hydrocarbon oils, esters, ethers, and the like, and mixtures thereof. The aqueous phase may comprise water. However, the aqueous phase may also comprise components different from water, including but not limited to water-soluble moisturizing agents, conditioning agents, anti-microbials, humectants and/or other water-soluble skin care actives. Examples of a humectant are glycerin and/or other polyols. Emulsions may also contain an emulsifier. Emulsifiers may be nonionic, anionic or cationic.

A wide variety of optional components/ingredients may be included in the cosmetic compositions. For example, the cosmetic compositions may include absorbents, abrasives, anti-caking agents, antifoaming agents, antimicrobial agents, binders, biological additives, buffering agents, bulking agents, chemical additives, cosmetic biocides, denaturants, cosmetic astringents, drug astringents, external analgesics, film formers, humectants, opacifying agents, fragrances, pigments, colourings, essential oils, skin sensates, emollients, skin soothing agents, skin healing agents, pH adjusters, plasticizers, preservatives, preservative enhancers, propellants, reducing agents, additional skin-conditioning agents, skin penetration enhancing agents, skin protectants, solvents, suspending agents, emulsifiers, thickening agents, solubilizing agents, sunscreens, sunblocks, ultraviolet light absorbers or scattering agents, sunless tanning agents, antioxidants and/or radical scavengers, chelating agents, sequestrants, anti-acne agents, antiinflammatory agents, antiandrogens, depilation agents, desquamation agents/exfoliants, organic hydroxy acids, vitamins and derivatives thereof, and natural extracts. Such other materials are known in the art. Nonexclusive examples of such materials are described in Harry's Cosmeticology, 7th Ed., Harry and Wilkinson (Hill Publishers, London 1982); in Pharmaceutical Dosage Forms-Disperse Systems; Lieberman, Rieger and Banker, Vols. 1 (1988) and 2 (1989); Marcel Decker, Inc.; in The Chemistry and Manufacture of Cosmetics, 2nd. Ed., deNavarre (Van Nostrand 1962-1965); and in The Handbook of Cosmetic Science and Technology, 1st Ed.. Knowlton and Pearce (Elsevier 1993). In a particularly preferred embodiment of the invention the cosmetic composition of the present invention is free from cortisone or any cortisone derivative.

In particular the following ingredients may be comprises in the cosmetic compositions of the present invention, wherein dependent from the particular type of composition the suitable ingredients can be selected. E.g. an detergent composition like a shampoo, a shower gel a liquid soap or similar comprises typically any surfactant, water and other suitable ingredients for the carrier, wherein a cream, lotion or body milk comprises typically oily and/or fatty components.

All the ingredients suitable for the particular composition type are known to persons skilled in the art. The following examples of ingredients may be comprised in the compositions of the invention:

### Oil/Oil bodies

Suitable oil bodies are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, such as, for example, myristyl myristate, myristyl palmitate, myristyl stearate, myristyl isostearate, myristyl oleate, myristyl be- henate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈-alkylhydroxy carboxylic acids with linear or branched C₆-C₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆-Ci₀-fatty acids, liquid mono-/di-/triglyceride mixtures based on C₆-C₁₈-fatty acids, esters of C₆-C₂₂- fatty alcohols and/or Guerbet alcohols with aromatic carboxylic acids, in particular benzoic acid, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol^{(R)} CC), Guerbet carbonates, based on fatty alcohols having 6 to 18, preferably 8 to 10, carbon atoms, esters of benzoic acid with linear and/or branched C₆-C₂₂-alcohols (e.g. Finsolv^{(R)} TN), linear or branched, symmetrical or asymmetrical dialkyl ethers having 6 to 22 carbon atoms per alkyl group, such as, for example, dicaprylyl ether (Cetiol^{(R)} OE), ring-opening products of epox- idized fatty acid esters with polyols, silicone oils (cyclomethicones, silicone methicone grades, etc.), aliphatic or naphthenic hydrocarbons, such as, for example, squalane, squa- lene or dialkylcyclohexanes, and/or mineral oils.

### Waxes

Among the group of suitable waxes one can differentiate between the following types:
• superfatting agents
• consistency factors
• pearlising waxes, and
▪ natural waxes

Superfatting agents. Superfatting agents may be selected from substances such as for example, lanolin and lecithin and also polyethoxylated or acylated lanolin and lecithin derivatives, polyol fatty acid esters, monoglycerides and fatty acid alkanolamides; fatty acid alkanolamides can also serve as foam stabilizers.

Consistency factors. The consistency factors can be for example fatty alcohols or hydroxyfatty alcohols containing 12 to 22 and preferably 16 to 18 carbon atoms and also partial glycerides, fatty acids or hydroxyfatty acids of the same carbon range. A combination of these substances with alkyl oligoglucosides and/or fatty acid N-methyl glucamides of the same chain length and/or polyglycerol poly-12-hydroxystea rates is preferably used.

Pearlising waxes. Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxysubstituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

Natural waxes. Besides natural oils used, waxes may also be present in the preparations, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, monta n ester waxes, sasol waxes, hydrogenated jojoba waxes and synthetic waxes such as, for example, polyalkylene waxes and polyethylene glycol waxes.

### Emulsifiers

The emulsifiers, detergents or surfactants may be of non-ionic, anionic, cationic and/or amphoteric nature.

In particular preferred are non-ionic emulsifiers, such as:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- 12/18 fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C₆/₂₂ fatty acids, ricinoleic acid and 12-hydroxystearic acid and glycerol, polyglycerol, pentaerythritol, dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆-₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/16} fatty acid monoesters and diesters of addition products of ethylene oxide onto glycerol are known as lipid layer enhancers for cosmetic formulations. The most preferred emulsifiers are described in more detail as follows:
Partial glycerides. Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

Sorbitan esters. Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

Polyglycerol esters. Typical examples of suitable polyglycerol esters are Polyglyceryl-2 Dipolyhydroxystearate (Dehymuls PGPH), Polyglycerin-3-Diisostearate (Lameform TGI), Polyglyceryl-4 Isostearate (Isolan GI 34), Polyglyceryl-3 Oleate, Diisostearoyl Polyglyceryl-3 Diisostearate (Isolan^{(R)} PDI), Polyglyceryl-3 Methylglucose Distearate (Tego Care 450), Polyglyceryl-3 Beeswax (Cera Bellina), Polyglyceryl-4 Caprate (Polyglycerol Caprate T2010/90), Polyglyceryl-3 Cetyl Ether (Chimexane N L), Polyglyceryl-3 Distearate (Cremophor^{(R)} GS 32) and Polyglyceryl Polyricinoleate (Admul WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

Anionic emulsifiers. Typical anionic emulsifiers are aliphatic C12-22 fatty acids, such as palmitic acid, stearic acid or behenic acid for example, and C12-22 dicarboxylic acids, such as azelaic acid or sebacic acid for example.

Amphotheric or zwitterionic emulsifiers. Other suitable emulsifiers are amphoteric or zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl- 3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of Cocamidopropyl Betaine is particularly preferred. Ampholytic surfactants are also suitable emulsifiers. Am pholytic surfactants are surface-active compounds which, in addition to a C₈/₁₈ alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N- alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C12/18 acyl sarcosine.

### Active principles

The compositions according to the present invention may contain additional ingredients encompassed by the term "active principles". Examples for suitable ingredients are abrasives, anti-acne agents, agents against ageing of the skin, anti-cellulitis agents, antidandruff agents, anti-inflammatory agents, irritation-preventing agents, irritation-inhibiting agents, antioxidants, astringents, perspiration-inhibiting agents, antiseptic agents, anti-statics, binders, buffers, carrier materials, chelating agents, cell stimulants, cleansing agents, care agents, depilatory agents, surface-active substances, deodorizing agents, antiperspirants, softeners, enzymes, essential oils, fibres, film-forming agents, fixatives, foam-forming agents, foam stabilizers, substances for preventing foaming, foam boosters, gelling agents, gel-forming agents, hair care agents, hair-setting agents, hair- straightening agents, moisture-donating agents, moisturizing substances, moisture-retaining substances, bleaching agents, strengthening agents, stain-removing agents, optically brightening agents, impregnating agents, dirt-repellent agents, friction-reducing agents, lubricants, moisturizing agents, opacifying agents, plasticizing agents, covering agents, polish, gloss agents, polymers, powders, proteins, re-oiling agents, abrading agents, silicones, skin-soothing agents, skin-cleansing agents, skin care agents, skin-healing agents, skin-lightening agents, skin-protecting agents, skin-softening agents, hair promotion agents, cooling agents, skin-cooling agents, warming agents, skin-warming agents, stabilizers, UV- absorbing agents, UV filters, detergents, fabric conditioning agents, suspending agents, skin- tanning agents, thickeners, vitamins, oils, waxes, fats, phospholipids, saturated fatty acids, mono- or polyunsaturated fatty acids, hydroxy acids, polyhydroxyfatty acids, liquefiers, dyestuffs, colour-protecting agents, pigments, anti-corrosives, aromas, flavouring substances, odoriferous substances, polyols, surfactants, electrolytes, organic solvents or silicone derivatives and the like as additional auxiliaries and additives.

### Thickening agents and rheology additives

Suitable thickeners are polymeric thickeners, such as Aerosil^{(R)} types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols^{(R)} [Goodrich] or Synthalens^{(R)} [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### Polymers

Suitable cationic polymers are, for example, cationic cellulose derivatives such as, for example, the quaternized hydroxyethyl cellulose obtainable from Amerchol under the name of Polymer JR 400^{(R)}, cationic starch, copolymers of diallyl ammonium salts and acrylamides, quaternized vinyl pyrrolidone/vinyl imidazole polymers such as, for example, Luviquat^{(R)} (BASF), condensation products of polyglycols and amines, quaternized collagen polypeptides such as, for example, Lauryldimonium Hydroxypropyl Hydrolyzed Collagen (Lamequat^{(R)} L, Grunau), quaternized wheat polypeptides, polyethyleneimine, cationic silicone polymers such as, for example, amodimethicone, copolymers of adipic acid and dimethylaminohydroxypropyl diethylenetriamine (Cartaretine, Sandoz), copolymers of acrylic acid with dimethyl diallyl ammonium chloride (Merquat 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-l,3-propane, cationic guar gum such as, for example, Jaguar CBS, Jaguar C-17, Jaguar C-16 of Celanese, quaternized ammonium salt polymers such as, for example, Mirapol A-15, Mirapol AD-1, Mirapol AZ-1 of Miranol and the various polyquaternium types (for example 6, 7, 32 or 37) which can be found in the market under the tradenames Rheocare CC or Ultragel 300.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### Silicones

Suitable silicone compounds are, for example, dimethylpolysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. A detailed overview of suitable volatile silicones can be found in Todd et al. in Cosm. Toil. 91, 27 (1976).

### Sun protection compounds

The compositions according to the invention may contain at least one UV- A filter and/or at least one UV-B filter and/or a broadband filter and/or at least one inorganic pigment.

Preferred cosmetic compositions, preferably topical formulations according to the present invention may comprise one, two, three or more sun protection factors selected from the group consisting of 4-aminobenzoic acid and derivatives, salicylic acid derivatives, ben-zophenone derivatives, dibenzoylmethane derivatives, diphenyl acrylates, 3-imidazol-4-yl acrylic acid and esters thereof, benzofuran derivatives, benzylidene malonate derivatives, polymeric UV absorbers containing one or more organosilicon radicals, cinnamic acid deriva- tives, camphor derivatives, trianilino-s-triazine derivatives, 2-hydroxyphenylbenzotriazole derivatives, phenylbenzimidazole sulfonic acid derivatives and salts thereof, anthranilic acid menthyl esters, benzotriazole derivatives and indole derivatives.

Besides the groups of primary sun protection factors mentioned above, secondary sun protection factors of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L- carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophe- none, uric acid and derivatives thereof, mannose and derivatives thereof, superoxide dismutase, titanium dioxide (for example dispersions in ethanol), zinc and derivatives thereof (for example ZnO, ZnS0₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

Advantageous inorganic secondary light protection pigments are finely dispersed metal oxides and metal salts which are also mentioned in WO 2005 123101 A1. Also preferred are particulate UV filters or inorganic pigments, which can optionally be hydrophobed, can be used, such as the oxides of titanium (Ti0₂), zinc (ZnO), iron (Fe₂0₃), zirconium (Zr0₂), silicon (Si0₂), manganese (e.g. MnO), aluminium (Al₂0₃), cerium (e.g. Ce₂0₃) and/or mixtures thereof.

### Anti-ageing agents

Anti-ageing or biogenic agents are, for example antioxidants, matrix-metalloproteinase inhibitors (M M PI), skin moisturizing agents, glycosaminglycan stimulators, anti-inflammatory agents, TRPV1 antagonists and plant extracts. Antioxidants, amino acids (preferably glycine, histidine, tyrosine, tryptophane) and derivatives thereof, imidazoles (preferably urocanic acid) and derivatives thereof, peptides, preferably D,L-carnosine, D-carnosine, L-carnosine and derivatives thereof (preferably anserine), carnitine, creatine, matrikine peptides (preferably lysyl-threonyl-threonyl-lysyl-serine) and palmitoylated pentapeptides, carotenoids, carotenes (preferably alpha-carotene, beta- carotene, lycopene) and derivatives thereof, lipoic acid and derivatives thereof (preferably dihydrolipoic acid), aurothioglucose, propyl thiouracil and other thiols (preferably thioredoxine, glutathione, cysteine, cystine, cystamine and glycosyl, N-acetyl, methyl, ethyl, propyl, amyl, butyl and lauryl, palmitoyl, oleyl, gamma-linoleyl, cholesteryl, glyceryl and oligoglyceryl esters thereof) and salts thereof, dilauryl thiodipropionate, distearyl thiodipropionate, thiodipropionic acid and derivatives thereof (preferably esters, ethers, peptides, lipids, nucleotides, nucleosides and salts) and sulfoximine compounds (preferably buthionine sulfoximines, homocysteine sulfoximine, buthionine sulfones, penta-, hexa-, heptathionine sulfoximine) in very small tolerated doses (e.g. pmol to micromole/ kg), also (metal) chelators (preferably alpha-hydroxy fatty acids, palmitic acid, phytic acid, lactoferrin, alpha-hydroxy acids (preferably citric acid, lactic acid, malic acid), humic acid, bile acid, bile extracts, tannins, bilirubin, biliverdin, EDTA, EGTA and derivatives thereof), unsaturated fatty acids and derivatives thereof (preferably gamma-linolenic acid, linoleic acid, oleic acid), folic acid and derivatives thereof, ubiquinone and derivatives thereof, ubiquinol and derivatives thereof, vitamin C and derivatives (preferably ascorbyl palmitate, Mg ascorbyl phosphate, ascorbyl acetate, ascorbyl glucoside), tocopherols and derivatives (preferably vitamin E acetate), vitamin A and derivatives (vitamin A palmitate) and coniferyl benzoate of benzoic resin, rutinic acid and derivatives thereof, flavonoids and glycosylated precursors thereof, in particular quercetin and derivatives thereof, preferably alpha-glucosyl rutin, rosmarinic acid, carnosol, carnosolic acid, resveratrol, caffeic acid and derivatives thereof, sinapic acid and derivatives thereof, ferulic acid and derivatives thereof, curcuminoids, chlorogenic acid and derivatives thereof, retinoids, preferably retinyl palmitate, retinol or tretinoin, ursolic acid, levulinic acid, butyl hydroxytoluene, butyl hydroxyanisole, nordihydroguaiac acid, nordihydroguaiaretic acid, trihydroxybutyrophenone, uric acid and derivatives thereof, mannose and derivatives thereof, zinc and derivatives thereof (preferably ZnO, ZnS0₄), selenium and derivatives thereof (preferably selenium methionine), superoxide dismutase, stilbenes and derivatives thereof (preferably stilbene oxide, trans-stilbene oxide) and the derivatives (salts, esters, ethers, sugars, nucleotides, nucleosides, peptides and lipids) of these cited active ingredients which are suitable according to the invention or extracts or fractions of plants having an antioxidant effect, preferably green tea, rooibos, honeybush, grape, rosemary, sage, melissa, thyme, lavender, olive, oats, cocoa, ginkgo, ginseng, liquorice, honeysuckle, sophora, pueraria, pinus, citrus, Phyllanthus emblica or St. John's wort, grape seeds, wheat germ, Phyllanthus emblica, coenzymes, preferably coenzyme Q.10, plastoquinone and menaquinone. Preferred antioxidants are selected from the group consisting of vitamin A and derivatives, vitamin C and derivatives, tocopherol and derivatives, preferably tocopheryl acetate, and ubiquinone.

### Skin-moisturizing agents

Preferred skin moisturizing agents are selected from the group consisting of alkane diols or alkane triols comprising 3 to 12 carbon atoms, preferably C₃-C₁₀-alkane diols and C₃-C₁₀-alkane triols. More preferably the skin moisturizing agents are selected from the group consisting of: glycerol, 1,2-propylene glycol, 1,2-butylene glycol, 1,3-butylene glycol, 1,2-pentanediol, 1,2-hexanediol, 1,2-octanediol and 1,2-decanediol.

### Glycosaminoglycan stimulators

Cosmetic compositions may comprise substances stimulating the synthesis of glycosaminoglycans selected from the group consisting of hyaluronic acid and derivatives or salts, Subliskin (Sederma, INCI: Sinorhizobium Meliloti Ferment Filtrate, Cetyl Hydroxyethylcellulose, Lecithin), Hyalufix (BASF, I NCI: Water, Butylene Glycol, Alpinia galanga leaf extract, Xanthan Gum, Caprylic/Capric Triglyceride), Stimulhyal (Soliance, I NCI: Calcium ketogluconate), Syn-Glycan (DSM, I NCI: Tetradecyl Aminobutyroylvalylaminobutyric Urea Trifluoroacetate, Glycerin, Magnesium chloride), Kalpariane (Biotech Marine), DC Upregulex (Distinctive Cosmetic Ingredients, INCI: Water, Butylene Glycol, Phospholipids, Hydrolyzed Sericin), glucosamine, N-acetyl glucosamine, retinoids, preferably retinol and vitamin A, Arctium lappa fruit extract, Eriobotrya japonica extract, Genkwanin, N-Methyl-L-serine, (-)-alpha-bisabolol or synthetic alpha-bisabolol such as e.g. Dragosantol and Dragosantol 100 from Symrise, oat glucan, Echinacea purpurea extract and soy protein hydrolysate. Preferred actives are selected from the group consisting of hyaluronic acid and derivatives or salts, retinol and derivatives, (-)-alpha-bisabolol or synthetic alpha-bisabolol such as e.g. Dragosantol and Dragosantol 100 from Symrise, oat glucan, Echinacea purpurea extract, Sinorhizobium Meliloti Ferment Filtrate, Calcium ketogluconate, Alpinia galanga leaf extract and tetradecyl aminobutyroylvalylaminobutyric urea trifluoroacetate.

### Anti-inflammatory agents

The compositions may also comprise anti-inflammatory and/or redness and/or itch ameliorating ingredients, wherein it particularly preferred to use naturally occurring agents. Natural or naturally occurring anti-inflammatory compounds or mixtures of compounds that alleviate reddening and/or itching are in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea; preferably selected from the group consisting of extracts or fractions from camomile, Aloe vera, oats, calendula, arnica, honeysuckle, rosemary, witch hazel, ginger or Echinacea, and/or pure substances, preferably alpha-bisabolol, apigenin, apigenin-7-glucoside, gingerols, shogaols, gingerdiols, dehydrogingerdiones, paradols, natural or naturally occuring avenanthramides, preferably tranilast, avenanthramide A, avenanthramide B, avenanthramide C, non-natural or non-naturally occuring avenanthramides, preferably dihydroavenanthramide D, dihydroavenanthramide E, avenanthramide D, avenanthramide E, avenanthramide F, boswellic acid, phytosterols, glycyrrhizin, glabridin and licochalcone A; preferably selected from the group consisting of alpha-bisabolol, natural avenanthramides, non-natural avenanthramides, preferably dihydroavenanthramide D (as described in WO 2004 047833 A1), phytosterols, glycyrrhizin, and licochalcone A, and/or panthenol, lanolin, (pseudo-)ceramides [preferably Ceramide 2, hydroxypropyl bispalmitamide MEA, cetyloxypropyl glyceryl methoxypropyl myristamide, N-(1- hexadecanoyl)-4-hydroxy-L-proline (1-hexadecyl) ester, hydroxyethyl palmityl oxyhydroxypropyl palmitamide], glycosphingolipids, phytosterols, chitosan, mannose, lactose and beta-glucans, in particular 1,3-1,4- - glucan from oats.

Anti-cellulite agents. Anti-cellulite agents and lipolytic agents are preferably selected from the group consisting of those described in WO 2007/077541, and beta-adrenergic receptor agonists such as synephrine and its derivatives, and cyclohexyl carbamates described in WO 2010/097479. Agents enhancing or boosting the activity of anti-cellulite agents, in particular agents which stimulate and/or depolarise C nerve fibres, are preferably selected from the group consisting of capsaicin and derivatives thereof, vanillyl-nonylamid and derivatives thereof, L-carnitine, coenzym A, isoflavonoides, soy extracts, ananas extract and conjugated linoleic acid.

### Fat enhancing agents

Formulations and products according to the present invention may also comprise one or more fat enhancing and/or adipogenic agents as well as agents enhancing or boosting the activity of fat enhancing agents. A fat enhancing agent is for example hydroxymethoxyphenyl propylmethylmethoxybenzofuran (trade name: Sym3D^{(R)}).

### Hair growth activators or inhibitors

Formulations and products according to the present invention may also comprise one or more hair growth activators, i.e. agents to stimulate hair growth. Hair growth activators are preferably selected from the group consisting of pyrimidine derivatives such as 2,4- diaminopyrimidine-3-oxide (Aminexil), 2,4-diamino-6-piperidinopyrimidine-3-oxide (Minoxidil) and derivatives thereof, 6-amino-l,2-dihydro-l-hydroxy-2-imino-4-piperidinopyrimidine and its derivatives, xanthine alkaloids such as caffeine, theobromine and theophylline and derivatives thereof, quercetin and derivatives, dihydroquercetin (taxifolin) and derivatives, potassium channel openers, antiandrogenic agents, synthetic or natural 5-reductase inhibitors, nicotinic acid esters such as tocopheryl nicotinate, benzyl nicotinate and C₁-C₆ alkyl nicotinate, proteins such as for example the tripeptide Lys-Pro-Val, diphencypren, hormons, finasteride, dutasteride, flutamide, bicalutamide, pregnane derivatives, progesterone and its derivatives, cyproterone acetate, spironolactone and other diuretics, calcineurin inhibitors such as FK506 (Tacrolimus, Fujimycin) and its derivatives, Cyclosporin A and derivatives thereof, zinc and zinc salts, polyphenols, procyanidins, proanthocyanidins, phytosterols such as for example beta-sitosterol, biotin, eugenol, (plus or minus)-beta-citronellol, panthenol, glycogen for example from mussels, extracts from microorganisms, algae, plants and plant parts of for example the genera dandelion (Leontodon or Taraxacum), Orthosiphon, Vitex, Coffea, Paullinia, Theobroma, Asiasarum, Cucurbita or Styphnolobium, Serenoa repens (saw palmetto), Sophora flavescens, Pygeum africanum, Panicum miliaceum, Cimicifuga racemosa, Glycine max, Eugenia caryophyllata, Cotinus coggygria, Hibiscus rosa-sinensis, Camellia sinensis, Ilex paraguariensis, Isochrysis galbana, licorice, grape, apple, barley or hops or/and hydrolysates from rice or wheat.

Alternatively, formulations and products according to the present invention may comprise one or more hair growth inhibitors (as described above), i.e. agents to reduce or prevent hair growth. Hair growth inhibitors are preferably selected from the group consisting of activin, activin derivatives or activin agonists, ornithine decarboxylase inhibitors such as alpha-difluoromethylornithine or pentacyclic triterpenes like for example ursolic acid, betulin, betulinic acid, oleanolic acid and derivatives thereof, 5alpha-reductase inhibitors, androgen receptor antagonists, S-adenosylmethionine decarboxylase inhibitors, gamma- glutamyl transpeptidase inhibitors, transglutaminase inhibitors, soybean-derived serine protease inhibitors, extracts from microorganisms, algae, different microalgae or plants and plant parts of for example the families Leguminosae, Solanaceae, Graminae, Asclepiadaceae or Cucurbitaceae, the genera Chondrus, Gloiopeltis, Ceramium, Durvillea, Glycine max, Sanguisorba officinalis, Calendula officinalis, Hamamelis virginiana, Arnica montana, Salix alba, Hypericum perforatum or Gymnema sylvestre.

### Cooling agents

The compositions may also contain one or more substances with a physiological cooling effect (cooling agents), which are preferably selected here from the following list: menthol and menthol derivatives (for example L-menthol, D-menthol, racemic menthol, isomenthol, neoisomenthol, neomenthol) menthylethers (for example (I-menthoxy)-1,2-propandiol, (I-menthoxy)-2-methyl-1,2-propandiol, I-menthyl-methylether), menthylesters (for example menthylformiate, menthylacetate, menthylisobutyrate, menthyllactates, L- menthyl-L-lactate, L-menthyl-D-lactate, menthyl-(2-methoxy)acetate, menthyl-(2-methoxyethoxy)acetate, menthylpyroglutamate), menthylcarbonates (for example menthylpropyleneglycolcarbonate, menthylethyleneglycolcarbonate, menthylglycerolcarbonate or mixtures thereof), the semi-esters of menthols with a dicarboxylic acid or derivatives thereof (for example mono-menthylsuccinate, mono-menthylglutarate, mono-menthylmalonate, O-menthyl succinic acid ester-N,N-(dimethyl)amide, O-menthyl succinic acid ester amide), menthanecarboxylic acid amides (in this case preferably menthanecarboxylic acid-N-ethylamide or Na-(menthanecarbonyl)glycinethylester as described in US 4,150,052, menthanecarboxylic acid-N-(4-cyanophenyl)amide or menthanecarboxylic acid-N-(4-cyanomethylphenyl)amide as described in WO 2005 049553 A1, methanecarboxylic acid-N-(alkoxyalkyl)amides), menthone and menthone derivatives (for example L-menthone glycerol ketal), 2,3-dimethyl-2-(2-propyl)-butyric acid derivatives (for example 2,3-dimethyl-2-(2-propyl)-butyric acid-N-methylamide), isopulegol or its esters (I-(-)-isopulegol, I-(-)-isopulegolacetate), menthane derivatives (for example p- menthane-3,8-diol), cubebol or synthetic or natural mixtures, containing cubebol, pyrrolidone derivatives of cycloalkyldione derivatives (for example 3-methyl-2(I-pyrrolidinyl)-2-cyclopentene-I-one) or tetrahydropyrimidine-2-one (for example iciline or related compounds, as described in WO 2004/026840), further carboxamides (for example N-(2-(pyridin-2-yl)ethyl)-3-p-menthanecarboxamide or related compounds), (IR,2S,5R)-N-(4- Methoxyphenyl)-5-methyl-2-(I-isopropyl)cyclohexane-carboxamide, oxamates (preferably those described in EP 2033688 A2).

### Anti-microbial agents

Suitable anti-microbial agents are, in principle, all substances effective against Gram- positive bacteria, such as, for example, 4- hydroxybenzoic acid and its salts and esters, N-(4- chlorophenyl)-N'-(3,4- dichlorophenyl)urea, 2,4,4'-trichloro-2'-hydroxy-diphenyl ether (triclosan), 4-chloro-3,5-dimethyl-phenol, 2,2'-methylenebis(6-bromo-4- chlorophenol), 3- methyl-4-(I-methylethyl)phenol, 2-benzyl-4-chloro-phenol, 3-(4-chlorophenoxy)-I,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GM L), diglycerol monocaprate (DMC), salicylic acid N-alkylamides, such as, for example, n-octylsalicylamide or n-decylsalicylamide.

### Enzyme inhibitors

Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen CAT). The substances inhibit enzyme activity, thereby reducing the formation of odour. Other substances which are suitable esterase inhibitors are sterol sulfates or phosphates, such as, for example, lanosterol, cholesterol, campesterol, stigmasterol and sitosterol sulfate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

### Odour absorbers and antiperspirant active agents

Suitable odour absorbers are substances which are able to absorb and largely retain odour-forming compounds. They lower the partial pressure of the individual components, thus also reducing their rate of diffusion. It is important that perfumes must remain unimpaired in this process. Odour absorbers are not effective against bacteria. They comprise, for example, as main constituent, a complex zinc salt of ricinoleic acid or specific, largely odour-neutral fragrances which are known to the person skilled in the art as "fixatives", such as, for example, extracts of labdanum or styrax or certain abietic acid derivatives. The odour masking agents are fragrances or perfume oils, which, in addition to their function as odour masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal products, such as, for example, civet and castoreum. Typical synthetic fragrance compounds are products of the ester, ether, aldehyde, ketone, alcohol, and hydrocarbon type. Fragrance compounds of the ester type are, for example, benzyl acetate, p-tert- butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linaool, phenylethyl alcohol and terpineol, and the hydrocarbons include mainly the terpenes and balsams. Preference is, however, given to using mixtures of different fragrances which together produce a pleasing fragrance note. Essential oils of relatively low volatility, which are mostly used as aroma components, are also suitable as perfume oils, e.g. sage oil, camomile oil, oil of cloves, melissa oil, mint oil, cinnamon leaf oil, linden flower oil, juniperberry oil, vetiver oil, olibanum oil, galbanum oil, labdanum oil and lavandin oil. Preference is given to using bergamot oil, dihydromyrcenol, lilial, lyral, citronellol, phenylethyl alcohol, a-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, beta-damascone, geranium oil bourbon, cyclohexyl salicylate, Vertofix coeur, iso-E-super, Fixolide N P, evernyl, iraldein gamma, phenylacetic acid, geranyl acetate, benzyl acetate, rose oxide, romilat, irotyl and floramat alone or in mixtures.

Suitable astringent antiperspirant active ingredients are primarily salts of aluminium, zirconium or of zinc. Such suitable antihydrotic active ingredients are, for example, aluminium chloride, aluminium chlorohydrate, aluminium dichlorohydrate, aluminium sesquichlorohydrate and complex compounds thereof, e.g. with 1,2- propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, e.g. with amino acids, such as glycine.

### Film formers and anti-dandruff agents

Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

Suitable antidandruff agents are Pirocton Olamin (1-hydroxy-4-methyl-6-(2,4,4- trimethylpentyl)-2-(1H)-pyridinone monoethanolamine salt), Baypival (Climbazole), Ketoconazol^{(R)} (4-acetyl-I-{4-[2-(2,4-dichlorophenyl)r-2-(1H-imidazol-I-ylmethyl)-1,3-dioxylan-c-4-ylmethoxyphenyl}-piperazine, ketoconazole, elubiol, selenium disulfide, colloidal sulfur, sulfur polyethylene glycol sorbitan monooleate, sulfur ricinol polyethoxylate, sulfur tar distillate, salicylic acid (or in combination with hexachlorophene), undecylenic acid, monoeth- anolamide sulfosuccinate Na salt, Lamepon UD (protein/undecylenic acid condensate), zinc pyrithione, aluminium pyrithione and magnesium pyrithione/dipyrithione magnesium sulfate.

### Carriers and Hydrotropes

Preferred cosmetics carrier materials are solid or liquid at 20 °C and 1013 mbar (including highly viscous substances) as for example glycerol, 1,2-propylene glycol, 1,2-butylene glycol, 1,3-propylene glycol, 1,3-butylene glycol, ethanol, water and mixtures of two or more of said liquid carrier materials with water. Optionally, these preparations according to the invention may be produced using preservatives or solubilizers. Other preferred liquid carrier substances, which may be a component of a preparation according to the invention are selected from the group consisting of oils such as vegetable oil, neutral oil and mineral oil.

Preferred solid carrier materials, which may be a component of a composition according to the invention are hydrocolloids, such as starches, degraded starches, chemically or physically modified starches, dextrins, (powdery) maltodextrins (preferably with a dextrose equivalent value of 5 to 25, preferably of 10 -20), lactose, silicon dioxide, glucose, modified celluloses, gum arabic, ghatti gum, traganth, karaya, carrageenan, pullulan, curdlan, xanthan gum, gellan gum, guar flour, carob bean flour, alginates, agar, pectin and inulin and mixtures of two or more of these solids, in particular maltodextrins (preferably with a dextrose equivalent value of 15 - 20), lactose, silicon dioxide and/or glucose.

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average mo- lecular weight of 100 to 1000 Dalton;
- technical oligoglycerol mixtures with a degree of self-condensation of 1.5 to 10, such as for example technical diglycerol mixtures with a diglycerol content of 40 to 50 percent by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines, such as diethanolamine or 2-aminopropane-I,3-diol.

### Preservatives

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive").

### Perfume oils and fragrances

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert. butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetalde- hyde, cyclamen aldehyde, hydroxycitronellal, lilial and bourgeonal. Examples of suitable ketones are the ionones, isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide N P, evernyl, iraldein gamma, phenylacetic acid, geranyl acetate, benzyl acetate, rose oxide, romillat, irotyl and floramat.

### Detergents/surfactants

Detergents or surfactants usually are present in cosmetic compositions used and suitable for cleaning the skin or body of a mammal. Examples hereof are rinse-off products like shampoo, shower gels, hair conditioner, liquid soap, barred soap or similar.
Suitable detergents are non-ionic surfactants, anionic surfactants, cationic surfactants or amphoteric surfactants. All these type of surfactants suitable for body or hair cleaning are known in the art. Examples for such surfactants are listed below:
Among the numerous cationic surface active agents which may be used are distearyl dimethyl ammonium chloride, stearyl dimethyl benzyl ammonium chloride, stearyl trimethyl ammonium chloride, coco dimethyl benzyl ammonium chloride, dicoco dimethyl ammonium chloride, cetyl pyridinium chloride, cetyl trimethyl ammonium bromide, stearyl amine salts that are soluble in water such as stearyl amine acetate and stearyl amine hydrochloride, stearyl dimethyl amine hydrochloride, distearyl amine hydrochloride, alkyl phenoxyethoxyethyl dimethyl ammonium chloride, decyl pyridinium bromide, pyridinium chloride derivative of the acetyl amino ethyl esters of lauric acid, lauryl trimethyl ammonium chloride, decyl amine acetate, lauryl dimethyl ethyl ammonium chloride, the lactic acid and citric acid and other acid salts of stearyl-1-amido- ethyl-2-heptadecyl-2-imidazoline, quaternaries of such imizadoline with methyl chloride, benzyl chloride, chloroacetic acid and similar compounds, mixtures of the foregoing, and the like. Particularly preferred are stearyl trimethyl ammonium chloride, the lactic or the citric acid salt of stearyl-I-amidoethyl-2-heptadecyl-2-imidazoline, or mixtures thereof.

Any type of anionic surfactant may be used. It is preferable that the anionic surfactant be selected from the group consisting of (C₆-C₃₀) alkyl sulfates, (C₆-C₃₀) alkyl ether sulfates, (C₆-C₃₀) alkylamido ether sulfates, alkylaryl polyether sulfates, monoglyceride sulfates; (C₆-C₃₀) alkylsulfonates, (C₆-C₃₀) alkylamide sulfonates, (C₆-C₃₀) alkylaryl sulfonates, α-olefin sulfonates, paraffin sulfonates; (C₆-C₃₀) alkyl phosphates; (C₆-C₃₀) alkyl sulfoacetates; (C₆-C₂₀)acyl sarcosinates; (C₆-C₃₀) alkylpolyglycoside carboxylic ethers; (C₆-C₃₀) alkylpolyglycoside sulfosuccinates; (C₆-C₃₀) alkyl sulfosuccinamates; (C₆-C₂₄)acyl isethionates; N- (C₆-C₂₄) acyl taurates; C₆-C₃₀ fatty acid salts; coconut oil acid salts or hydrogenated coconut oil acid salts; (C₈-C₂₀)acyl lactylates; (C₆-C₃₀) alkyl-D-galactoside uronic acid salts; and corresponding acids.

The amphoteric surfactants may not be limited specifically. The amphoteric surfactants may be, for example, amine derivatives such as aliphatic secondary or tertiary amine, and optionally quaternized amine derivatives, in which the aliphatic radical is a linear or branched chain comprising 8 to 22 carbon atoms and comprising at least one water-solubilizing anionic group (for example, carboxylate, sulphonate, sulphate, phosphate or phosphonate). The amphoteric surfactant may preferably be selected from the group consisting of betaines and amidoaminecarboxylated derivatives.

The betaine-type amphoteric surfactant is preferably selected from the group consisting of alkylbetaines, alkylamidoalkylbetaines, sulfobetaines, phosphobetaines, and alkylamidoalkylsulfobetaines, in particular, (C₅-C₂₄) alkylbetaines, (C₈-C₂₀) alkylamido (C₁-C₈) alkylbetaines, sulphobetaines, and (C₈-C₂₄) alkylamido (C₁-C₈) alkylsulphobetaines. In one embodiment, the amphoteric surfactants of betaine type are chosen from (C₈-C₂₄) alkylbetaines, (C₅-C₂₄) alkylamido (C₁-C₈) alkylsulphobetaines, sulphobetaines, and phosphobetaines.

Examples of amphoteric surfactant are known under the names cocobetaine, laurylbetaine, cetylbetaine, coco/oleamidopropylbetaine, cocamidopropylbetaine, palmitamidopropylbetaine, stearamidopropylbetaine, cocamidoethylbetaine, cocamidopropylhydroxysultaine, oleamidopropylhydroxysultaine, cocohydroxysultaine, laurylhydroxysultaine, and cocosultaine, and may be used alone or as mixtures.

Among the amidoaminecarboxylated derivatives, mention may be made of the products sold under the name Miranol, as described in U.S. Pat. Nos. 2,528,378 and 2,781,354 and classified in the CTFA dictionary, 3rd edition, 1982, under the names Amphocarboxyglycinates and Amphocarboxypropionates, with the respective structures:
R1-CONHCH₂CH₂-N⁺ (R2) (R3) (CH₂COO-) in which:
R1 denotes an alkyl radical of an acid R1-COOH present in hydrolysed coconut oil, a heptyl, nonyl or undecyl radical, R2 denotes a beta-hydroxyethyl group, and
R3 denotes a carboxymethyl group; and
R1'-CONHCH₂CH₂-N (B) (C) in which:
B represents -CH₂CH₂OX',
C represents -(CH₂)_{Z}-Y', with z=1 or 2,
X' denotes a -CH₂CH₂-COOH group, -CH₂-COOZ', -CH₂CH₂-COOH, -CH₂CH₂-COOZ' or a hydrogen atom,
Y' denotes -COOH, -COOZ', -CH₂-CHOH-S0₃Z' or a -CH₂-CHOH-S0₃H radical,
Z' represents an ion of an alkaline or alkaline earth metal such as sodium, an ammonium ion or an ion derived from an organic amine, and
R1' denotes an alkyl radical of an acid R1'-COOH present in coconut oil or in hydrolysed linseed oil, an alkyl radical, such as a C₇, C₉, C₁₁ or C₁₃ alkyl radical, a C₁₇ alkyl radical and its isoform, or an unsaturated C₁₇ radical.

Preferably, the amphoteric surfactant chosen from amidoaminecarboxylated derivatives may be selected from the group consisting of (C₈-C₂₄) -alkyl amphomonoacetate, (C₈-C₂₄) alkyl amphodiacetate, (C₈-C₂₄) alkyl amphomonopropionates, and (C₈-C₂₄) alkyl amphodipropionates. These compounds are classified in the CTFA dictionary, 5th edition, 1993, under the names Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphopropionate, Disodium Caprylamphodipropionate, Disodium Caprylamphodipropionate, Lauroamphodipropionic acid and Cocoamphodipropionic acid.

The nonionic surfactants themselves are also compounds which are well known per se (in this respect see especially the "Handbook of Surfactants" by M. R. Porter, published by Blackie and Son (Glasgow and London), 1991, pp. 116-1 78). Thus, they can, for example, be chosen from alcohols, alpha-diols, alkylphenols and esters of fatty acids that are polyethoxylated, polypropoxylated or polyglycerolated and have at least one fatty chain comprising, for example, from 8 to 18 carbon atoms, it being possible for the number of ethylene oxide or propylene oxide groups to range from 1 to 50, and for the number of glycerol groups to range from 1 to 30. Maltose derivatives may also be mentioned. Mention may also be made of copolymers of ethylene oxide and/or of propylene oxide; condensates of ethylene oxide and/or of propylene oxide with fatty. alcohols; polyethoxylated fatty amides comprising, for example, from 2 to 30 mol of ethylene oxide; polyglycerolated fatty amides comprising, for example, from 1 to 5 glycerol groups, such as from 1.5 to 4; ethoxylated fatty acid esters of sorbitan comprising from 2 to 30 mol of ethylene oxide; ethoxylated oils from plant origin; fatty acid esters of sucrose; fatty acid esters of polyethylene glycol; fatty acid mono or diesters of glycerol; (C₆-C₂₄) alkylpolyglycosides; N- (C₆-C₂₄) alkylglucamine derivatives, amine oxides such as (C₁₀-C₁₄) alkylamine oxides or N- (C₁₀-C₁₄) acylaminopropylmorpholine oxides; and mixtures thereof.

The nonionic surfactants may be selected from monooxyalkylenated or polyoxyalkylenated, monoglycerolated or polyglycerolated nonionic surfactants. The oxyalkylene units are more particularly oxyethylene or oxypropylene units, or a combination thereof, preferably oxyethylene units.

Examples of oxyalkylenated nonionic surfactants that may be mentioned include: oxyalkylenated (C₈-C₂₄) alkylphenols, saturated or unsaturated, linear or branched, oxyalkylenated C₈-C₃₀ alcohols, saturated or unsaturated, linear or branched, oxyalkylenated C₈-C₃₀ amides, esters of saturated or unsaturated, linear or branched, C₈-C₃₀ acids and of polyethylene glycols, polyoxyalkylenated esters of saturated or unsaturated, linear or branched, C₈-C₃₀ acids and of sorbitol, saturated or unsaturated, oxyalkylenated plant oils, condensates of ethylene oxide and/or of propylene oxide, inter alia, alone or as mixtures.

The surfactants may comprise a number of moles of ethylene oxide and/or of propylene oxide of from 1 to 100 and preferably from 2 to 50. Advantageously, the nonionic surfactants do not comprise any oxypropylene units.

In accordance the present invention, the oxyalkylenated nonionic surfactants are preferably chosen from oxyethylenated C₈-C₃₀ alcohols or ethoxylated fatty esters. It will be noted that alkylpolyglycosides constitute nonionic surfactants which are particularly well suited within the context of the present invention.

Among alkylpolyglucosides which may be used, mention may be made of caprylyl/capryl glucoside, palmkernel/coco glucoside, cetearyl glucoside, decyl glucoside, lauryl glucoside, coco-glucoside, arachidyl gludoside, C₁₂-₂₀ alkyl glucoside, C₁₀-₁₆ alkyl glucoside, myristyl glucoside, myristoyl ethyl glucoside, methyl coco-glucoside, tallowyl ethyl glucoside, undecyl glucoside, octyldodecyl glucoside, isostearyl glucoside, lauroyl ethyl gludoside, cocoyl ethyl glucoside, caproyl ethyl glucoside, and butyl glucoside, but not limited thereto.

All the above mentioned ingredients should be considered as examples for suitable, but optional ingredients of the cosmetic compositions of the present invention. All these additional components are not limiting the invention. Examples of ingredients preferably used in the present invention may be selected from one or more glyceryl ester and/or one or more polyvalent alcohol, wherein preferably the one or more glyceryl ester is selected from the group consisting of glyceryl stearate and glyceryl caprylate, and preferably the one or more polyvalent alcohol is selected from the group consisting of glycerol and pentylene glycerol.

One further ingredient particularly preferred for use in a cosmetic composition according to the invention is Laureth-9 (INCI name) also known as Hydroxyl polyethoxy dodecane, Oxypolyethoxydodecane, Dodecylpolyethyleneglycolether, Macrogol lauryl ether or 3,6,9,12,15,18,21,24,27-nonaoxaonatriacontan-1-ol (IUPAC name). Its chemical formula is C₃₀H₆₂O₁₀. The chemical structure of Laureth-9 is as following:

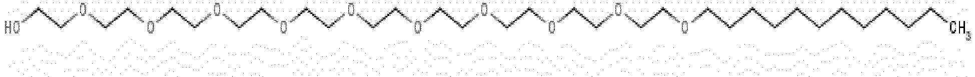

Laureth-9 is commonly used in cosmetic compositions as an antipruritic component and relieves therefore from itching caused by e.g. eczema and dry skin. For example, Laureth-9 is used in amounts of 3 to 5 wt.-% in several dermatological unguents/ointments and crèmes due to its antipruritic effect on skin. Furthermore, Laureth-9 is used in shampoos and wash lotions as a nonionic emulsifier and surfactant.

It has been found that the addition of Laureth-9 to the cosmetic composition of the present invention further increases the uniform distribution of the components Allantoin and/or Heliotropin. Laureth-9 serves as an emulgator, thus, the preparation of emulsions (e.g. cremes, lotions, emulsions, ointments, unguents, etc.) is supported. Further, Laureth-9 itself has a skin caring effect. Thus, in order to improve the distribution of Allantoin and Heliotropine in the cosmetic compositions even further, Laureth-9 may be added to said cosmetic compositions. It was found that the addition of Laureth-9 contributes to an even more consistent and stable distribution and incorporation of the further ingredients, like e.g. Allantoin and Heliotropine, in the cosmetic compositions.

In one embodiment of the invention, the cosmetic composition comprises Allantoin, Laureth-9 and ionically bound and/or free thiocyanate ions or derivatives thereof. In another embodiment of the invention, the cosmetic composition comprises Heliotropine, Laureth-9 and ionically bound and/or free thiocyanate ions or derivatives thereof. In a particular preferred embodiment, the cosmetic composition of the present invention comprises Allantoin, Heliotropine, Laureth-9 and ionically bound and/or free thiocyanate ions or derivatives thereof.

It is preferred according to the invention that the cosmetic composition of the present invention comprises Allantoin and ionically bound and/or free thiocyanate ions or derivatives thereof in a weight ratio of 10:1 to 1:40, preferably in a weight ratio of 5:1 to 1:20, more preferred in a weight ratio of 2:1 to 1:10, more preferred in a weight ratio of 1:1 to 1:8, more preferred in a weight ratio of 1:1 to 1:6, more preferred in a weight ratio of 1:2 to 1:5 and most preferred in a weight ratio of 1:2 to 1:4.

Further it is preferred that the cosmetic composition of the present invention comprises Heliotropine and ionically bound and/or free thiocyanate ions or derivatives thereof in a weight ratio of 10:1 to 1:40, preferably in a weight ratio of 5:1 to 1:20, more preferred in a weight ratio of 2:1 to 1:10, more preferred in a weight ratio of 1:1 to 1:8, more preferred in a weight ratio of 1:1 to 1:6, more preferred in a weight ratio of 1:1 to 1:5 and most preferred in a weight ratio of 1:1 to 1:4.

According to the present invention Allantoin and Heliotropine - independent from each other - each is present in the cosmetic composition in amounts of 0.01 to 5 wt.-% of the total weight of the composition, preferably in amounts of 0.02 to 3 wt.-% of the total weight of the composition, more preferred in amounts of 0.05 to 2 wt.-% of the total weight of the composition, even more preferred in amounts of 0.08 to 1 wt.-%, even more preferred 0.1 to 0.8 wt.-% and most preferred in amounts of 0.2 to 0.6 wt.-% or 0.2 to 0.4 wt.-% of the total weight of the composition. Thus, since both of the compounds are present in the composition, the above-mentioned ranges may be combined.

According to the present invention the ionically bound and/or free thiocyanate ions or derivatives thereof can be present in an amount of up to 5 wt.-% of the composition e.g. in an amount of 0.05 wt.-% to 4 wt.-% , 3 wt.-% , 2,.5 wt.-% , 2 wt.-% or 1.5 wt.-%, but preferably are present in the cosmetic composition in amounts of 0.1 to 1 wt.-% of the total weight of the composition, preferably in amounts of 0.1 to less than 1 wt.-%, e.g. in amounts of 0.1 to 0.98 wt.-% of the total weight of the composition, preferred in amounts of 0.2 to 0.95 wt.-% of the total weight of the composition, more preferred in amounts of 0.3 to 0.9 wt.-% of the total weight of the composition, more preferred in amounts of 0.4 to 0.85 wt.-% of the total weight of the composition, more preferred in amounts of 0.5 to 0.8 wt.-% of the total weight of the composition, even more preferred in amounts of 0.6 to 0.75 wt.-% of the total weight of the composition, and most preferred in amounts of 0.65 to 0.73 wt.-% of the total weight of the composition, (in particular when it is used as sodium thiocyanate).

The further preferred ingredient, Laureth-9 preferably is present in the cosmetic composition in amounts of 0.1 to 10 wt.-% of the total weight of the composition, preferably in amounts of 0.2 to 8 wt.-% of the total weight of the composition, more preferred in amounts of 0.5 to 6 wt.-% of the total weight of the composition, even more preferred in amounts of 0.75 to 5 wt.-% of the total weight of the composition and most preferred in amounts of 1 to 4 wt.-% of the total weight of the composition.

It has been found that the ingredients are particularly effective when they are added in amounts of the above mentioned ranges in a cosmetic composition.

The composition of the present invention can be formulated in any form that is applicable to the body, in particular to the skin or to the hair. For this purpose the compositions of the present invention can be formulated e.g. in form of creams, lotions, emulsions, body milks, body sprays, unguents, ointments and pastes, but further as well detergent compositions like shower gels, hair shampoo or conditioner, or antiperspirants (deodorizing compositions), without being limited to the mentioned.

In particular, the cosmetic compositions of the present invention are for topical use, especially for the care of irritated skin, wherein the irritation might be caused by any environment condition or disease, e.g. by dermatitis, neurodermitis, psoriasis, eczema, allergic reaction or by heat, cold, sunburn and/or dryness.

A further aspect of the present invention is a combination of Allantoin and Heliotropine, and ionically bound and/or free thiocyanate ions or derivatives thereof for use in the care and/or treatment of skin suffering from dermatitis, neurodermitis, psoriasis, eczema, allergic reaction, heat, cold, sunburn and/or dryness.

The present invention refers further to the use of ionically bound and/or free thiocyanate ions or derivatives thereof in cosmetic compositions for improved distribution and/or incorporation of Allantoin and Heliotropine in said cosmetic compositions.

### Examples

### Example 1:

A cosmetic composition according to the invention was prepared as follows:
An example base cream composition comprised (in wt.-%):

| | |
|---|---|
| Hydrogenated polydecene | 6.5 |
| Cetearyl alcohol | 5.5 |
| Starch derivative | 5 |
| Vegetable oil | 5 |
| Glyceryl searate | 3.6 |
| Glycerol | 3 |
| Emulsifier | 3 |
| Dimethicone | 2.5 |
| Isostearyl isostearate | 2 |
| Caprylic/capric triglyceride | 1 |
| Thickening agent | 0.8 |
| Plant extracts, essences | 0.8 - 2 |
| pH adjustment | 0.2 - 0.5 |
| Parfume, miscellous | 3 - 5 |
| Aqua | Ad 100 |

This base composition was used to include several desired skin caring compounds to increase the skin caring effect for irritated skin, in particular skin showing irritation from coditions like e.g. dermatitis, neurodermitis, psoriasis, eczema, or allergy.

In a first approach the intention was to include allantoin into such a cosmetic cream composition for topical application to the skin. Therefore 0.5 wt.-% allantoin was added to the composition in form of an oily solution. A smooth cream (A) was obtained. The cream was used daily over a time period of at least 6 weeks for skin care treatment. The test persons reported a very good caring effect at the beginning of the test period, however, a decreased caring effect over the time, wherein the caring effect appeared varying.

In an alternative approach 0.5 wt.-% heliotropine was added to the basic cream composition in form of an ehanolic solution, resulting in cream (B). Incorporation of the solution was somehow laborious, since the solution didn't combine very well with the cream body. Nevertheless, a creamy consistence was obtainable by intensive mixing. Again, the cream (B) was applied to irritated skin over a time period of at least 6 weeks. The skin caring effect of the cream was reported as being good over the time period.

In a further approach the base cream composition was supplemented with 0.5 wt.-% allantoin and 0.5 wt.-% heliotropin, resulting in composition (C). Incorporation of the components was possible by intensive mixing of the components, however, due to the mixing power a noticable amount of heat was applied to the cream. This is not desirable if instable ingredients are comprised in the desired cream, e.g. the allantoin. During the test period of 6 weeks the test persons reported about a very varying caring effect of the prepared product. During storage the consistence of the cream (C) became less smooth.

To the above approaches (A) or (B) additionally an amount of sodium thiocyanate was incorporated into the cream. In the composition comprising allantoin (A) as well as in the composition comprising heliotropin (B) different amounts of thiocyanate were tested: 4 wt.-%, 2 wt.-%, 1 wt.-%, 0.7 wt.-%, 0.5 wt.-%. As a first effect it was evident that the cream comprising heliotropine was easier to prepare, mainly independent from the concentration of the thiocyanate. The resulting creams were stored for a time period of 6 weeks and tested thereafter. Both compositions showed a stable consistency and good caring effect without variation of the caring effect. Even at the lowest concentration of the thiocyanate the cream remained stable in consistency and effectiveness during storage.

Furthermore, a composition according to composition (C) was supplemented with 0.7 wt.-% of thiocyanate, resulting in composition (D). During preparation it became clear that the components can be incorporated without any particular effort. Said composition (D) was tested again for a time period of 6 weeks with daily application to the skin of test persons. The test persons reported a very comfortable feeling during the whole test period with a very good caring effect without any noticable variation.

The cream was particularly smooth and comfortable to prepare when Laureth-9 was used as an emulsifier.

### Example 2

### Composition 1 (according to the invention):

In a cosmetic composition comprising mainly water, an oily / fatty body, Laureth-9 and further typical ingredients of a composition in form of a cream lotion for topical application, the following ingredients were included:

| | |
|---|---|
| Heliotropine: | 0.25 wt.-% |
| Allantoine: | 0.2 wt.-% |
| Sodium-Thiocyanate: | 0.7 wt.-% |

### Composition 2 (for comparison):

As a comparison example for the skin caring effect 0.25 wt.-% of hydrocortisone and 0.9 wt.- panthenol instead of heliotropine, allantoine and thiocyanate was added to the same basic composition.

Ten adult persons having neurodermitis used both of the compositions 1 and 2 for skin treatment over a time period of 28 days, wherein composition 1 was used at the left arm and composition 2 was used on the right arm. All of the test persons showed at least one of the following indications before treatment: redness, dryness and/or scaling of the skin. These parameters were considered for estimating the effect of the according composition on ameliorating the skin condition.

### I. Redness: on a scale of 1 to 100 the redness of the test area was determined:

**Table 1: left arm treatment with composition 1:**

| **Test person** | **Day 0** | **Day 9** | **Day 18** | **Day 28** | **% alteration** |
|---|---|---|---|---|---|
| 1 | 55.9 | 43.9 | 11.1 | 7.5 | - 86.60 |
| 2 | 63.6 | 50.5 | 11.8 | 11.7 | -81.60 |
| 3 | 63.4 | 36.1 | 5.7 | 3.1 | -95.10 |
| 4 | 48.8 | 38.0 | 12.9 | 0.0 | -100.0 |
| 5 | 54.5 | 39.5 | 13.1 | 14.7 | -73.0 |
| 6 | 35.8 | 25.5 | 11.2 | 1.7 | - 95.30 |
| 7 | 35.6 | 26.2 | 14.6 | 11.3 | -68.30 |
| 8 | 55.5 | 45.8 | 22.3 | 14.9 | - 73.20 |
| 9 | 30.9 | 21.1 | 16.1 | 13.7 | - 55.70 |
| 10 | 44.6 | 33.4 | 10.3 | 4.3 | - 90.40 |
| **average** | **48.9** | **36.0** | **12.9** | **8.3** | **- 83.03** |

**Table 2: right arm treatment with composition 2:**

| **Test person** | **Day 0** | **Day 9** | **Day 18** | **Day 28** | **% alteration** |
|---|---|---|---|---|---|
| 1 | 59.9 | 49.5 | 31.6 | 20.3 | -66.10 |
| 2 | 61.0 | 48.7 | 0.0 | 0.0 | -100.0 |
| 3 | 65.4 | 31.9 | 8.6 | 11.7 | -82.10 |
| 4 | 50.6 | 44.7 | 29.9 | 13.5 | - 73.30 |
| 5 | 52.8 | 42.9 | 17.8 | 14.9 | -71.80 |
| 6 | 37.3 | 28.4 | 11.2 | 0.0 | -100.0 |
| 7 | 41.1 | 30.1 | 25.0 | 15.8 | -61.10 |
| 8 | 52.7 | 49.4 | 25.2 | 17.0 | -67.70 |
| 9 | 29.6 | 25.3 | 13.2 | 6.9 | - 76.70 |
| 10 | 41.0 | 29.0 | 13.2 | 17.3 | - 57.80 |
| **average** | **49.1** | **38.0** | **17.6** | **11.7** | **- 76.17** |

### II. Dryness: on a scale of 1 to 100 the dryness of the test area was determined:

**Table 3: left arm treatment with composition 1:**

| **Test person** | **Day 0** | **Day 9** | **Day 18** | **Day 28** | **% alteration** |
|---|---|---|---|---|---|
| 1 | 67.3 | 49.0 | 21.8 | 6.7 | -90.0 |
| 2 | 59.3 | 24.7 | 0.0 | 4.8 | -91.90 |
| 3 | 57.2 | 26.1 | 0.0 | 0.0 | -100.0 |
| 4 | 69.2 | 46.6 | 35.0 | 20.8 | -69.90 |
| 5 | 52.5 | 38.3 | 11.1 | 6.1 | - 88.40 |
| 6 | 46.3 | 37.2 | 16.4 | 0.0 | -100.0 |
| 7 | 40.4 | 28.9 | 15.7 | 8.5 | -79.0 |
| 8 | 57.2 | 24.4 | 20.1 | 10.8 | -81.10 |
| 9 | 31.5 | 11.4 | 19.1 | 5.7 | -81.90 |
| 10 | 47.6 | 34.6 | 26.4 | 20.6 | - 56.70 |
| **average** | **52.9** | **32.1** | **16.6** | **8.4** | **- 84.12** |

**Table 4: right arm treatment with composition 2:**

| **Test person** | **Day 0** | **Day 9** | **Day 18** | **Day 28** | **% alteration** |
|---|---|---|---|---|---|
| 1 | 72.0 | 52.7 | 31.5 | 15.4 | - 78.60 |
| 2 | 71.2 | 50.9 | 0.0 | 0.0 | -100.0 |
| 3 | 54.2 | 30.2 | 10.5 | 0.0 | -100.0 |
| 4 | 64.5 | 52.8 | 25.4 | 11.2 | - 82.60 |
| 5 | 45.7 | 38.5 | 16.5 | 5.8 | - 87.30 |
| 6 | 42.9 | 25.4 | 12.9 | 6.8 | -84.10 |
| 7 | 53.8 | 34.4 | 10.9 | 4.3 | -92.0 |
| 8 | 45.3 | 35.5 | 27.6 | 21.6 | - 52.30 |
| 9 | 30.1 | 26.7 | 14.1 | 7.3 | - 75.70 |
| 10 | 42.5 | 28.3 | 14.4 | 0.0 | -100.0 |
| **average** | **52.2** | **36.5** | **16.4** | **7.2** | **- 86.21** |

### III. Scaling: on a scale of 1 to 100 the scaling of the test area was determined:

**Table 5: left arm treatment with composition 1:**

| **Test person** | **Day 0** | **Day 9** | **Day 18** | **Day 28** | **% alteration** |
|---|---|---|---|---|---|
| 1 | 19.2 | 0.0 | 0.0 | 0.0 | -100.0 |
| 2 | 64.7 | 21.9 | 0.0 | 0.0 | -100.0 |
| 3 | 60.7 | 18.8 | 0.0 | 0.0 | -100.0 |
| 4 | 0.0 | 0.0 | 0.0 | 0.0 | 0.0 |
| 5 | 55.1 | 46.2 | 9.7 | 7.5 | - 86.40 |
| 6 | 7.9 | 0.0 | 0.0 | 0.0 | -100.0 |
| 7 | 7.3 | 0.0 | 0.0 | 0.0 | -100.0 |
| 8 | 56.1 | 41.6 | 17.3 | 18.3 | - 67.40 |
| 9 | 30.4 | 9.8 | 5.1 | 8.7 | -71.40 |
| 10 | 10.4 | 0.0 | 0.0 | 0.0 | -100.0 |
| **average** | **31.2** | **13.8** | **3.2** | **3.5** | **- 88.78** |

**Table 6: right arm treatment with composition 2:**

| **Test person** | **Day 0** | **Day 9** | **Day 18** | **Day 28** | **% alteration** |
|---|---|---|---|---|---|
| 1 | 26.0 | 0.0 | 0.0 | 0.0 | -100.0 |
| 2 | 67.2 | 38.3 | 0.0 | 0.0 | -100.0 |
| 3 | 69.4 | 25.6 | 0.0 | 0.0 | -100.0 |
| 4 | 0.0 | 0.0 | 0.0 | 0.0 | 0.0 |
| 5 | 59.2 | 35.0 | 17.2 | 8.9 | - 85.00 |
| 6 | 7.1 | 0.0 | 0.0 | 0.0 | -100.0 |
| 7 | 9.6 | 0.0 | 0.0 | 0.0 | -100.0 |
| 8 | 58.3 | 35.4 | 31.2 | 18.8 | -67.80 |
| 9 | 31.6 | 22.7 | 9.8 | 5.5 | - 82.60 |
| 10 | 13.9 | 0.0 | 0.0 | 0.0 | -100.0 |
| **average** | **34.2** | **15.7** | **5.8** | **3.3** | **-90.35** |

The above shown results illustrate that the combination of the ingredients heliotropine, allantoine and thiocyanate have a similar ameliorating effect on irritated skin (considering three of the main skin conditions present when a patient has neurodermitis) as a composition including cortisone and panthenol. The treatment with cortisone is the standard treatment of skin irritated by neurodermitis, however, due to the side effects is sometimes not desired by the patients.

Further the test persons were asked to rate their subjective estimation of the parameters concerning the severity of their skin conditions. The results of the personal estimation on a scale of 0 to 7 (0= not present, 7= clearly present) are shown in table 7 as average values of estimation. The values in brackets after 28 days represent the % amelioration of the consideres symptoms compared to day 1.

**Table 7: average rating of skin conditions according personal estimation of the test persons:**

| **Composition /Day** | **Severity of disease** | **redness** | **dryness** | **itching** |
|---|---|---|---|---|
| Comp. 1/ Day 1 | 4 | 3.9 | 4 | 3.9 |
| Comp. 2/ Day 1 | 3.9 | 3.8 | 4 | 3.9 |
| Comp. 1/ Day 28 | 2 (-72.5%) | 2 (-71.8%) | 1.9 (72.5%) | 1.8 (-71.8&) |
| Comp. 2/ Day 28 | 2.3 (-66.7%) | 2.2 (-65.8%) | 2.1 (-70.0%) | 2.1 (-69.2%) |

## Claims

1. A cosmetic composition comprising:
a. Allantoin and Heliotropine and
b. ionically bound and/or free thiocyanate ions or derivatives thereof.

2. The cosmetic composition according to claim 1, wherein the thiocyanate ions are in form of alkali metal salts, ammonium salts or derivatives thereof, wherein preferably the alkali metal salts of thiocyanates are selected from sodium thiocyanate, potassium thiocyanate and/or ammonium thiocyanate, more preferably the alkali metal salts of thiocyanates are selected from sodium thiocyanate and potassium thiocyanate.

3. The cosmetic composition according any of the preceeding claims, wherein the composition further comprises Laureth-9.

4. The cosmetic composition according to any of the preceeding claims, wherein the composition further comprises one or more further ingredient of a cosmetic composition, selected from oil(s), wax(es), fatty acid(s), detergent(s), surfactant(s), emulsifier(s), particularly preferred at least a glyceryl ester and/or one or more polyvalent alcohol.

5. The cosmetic composition according to any of the preceeding claims, comprising Allantoin and ionically bound and/or free thiocyanate ions or derivatives thereof in a weight ratio of 10:1 to 1:40, preferably in a weight ratio of 5:1 to 1:20, more preferred in a weight ratio of 2:1 to 1:10 more preferred in a weight ratio of 1:1 to 1:8, more preferred in a weight ratio of 1:1 to 1:6, more preferred in a weight ratio of 1:2 to 1:5 and most preferred in a weight ratio of 1:2 to 1:4.

6. The cosmetic composition according to any of the preceeding claims, comprising Heliotropine and ionically bound and/or free thiocyanate ions or derivatives thereof in a weight ratio of 10:1 to 1:40, preferably in a weight ratio of 5:1 to 1:20, more preferred in a weight ratio of 2:1 to 1:10 and most preferred in a weight ratio of 1:1 to 1:4.

7. The cosmetic composition according to any of the preceeding claims, wherein Allantoin and/or Heliotropine independent from each other each is present in amounts of 0.01 to 5 wt.-% of the total weight of the composition, preferably in amounts of 0.02 to 3 wt.-% of the total weight of the composition, more preferred in amounts of 0.05 to 2 wt.-% of the total weight of the composition, even more preferred in amounts of 0.08 to 1 wt.-%, even more preferred 0.1 to 0.8 wt.-% and most preferred in amounts of 0.2 to 0.6 wt.-% of the total weight of the composition.

8. The cosmetic composition according to any of the preceeding claims, wherein ionically bound and/or free thiocyanate ions or derivatives thereof are present in amounts of 0.1 to 1 wt.-% of the total weight of the composition, preferably in amounts of 0.1 to less than 1 wt.-%, e.g. in amounts of 0.1 to 0.98 wt.-% of the total weight of the composition, preferred in amounts of 0.2 to 0.95 wt.-% of the total weight of the composition, more preferred in amounts of 0.3 to 0.9 wt.-% of the total weight of the composition, more preferred in amounts of 0.4 to 0.85 wt.-% of the total weight of the composition, more preferred in amounts of 0.5 to 0.8 wt.-% of the total weight of the composition, even more preferred in amounts of 0.6 to 0.75 wt.-% of the total weight of the composition, and most preferred in amounts of 0.65 to 0.73 wt.-% of the total weight of the composition.

9. The cosmetic composition according to any of the preceeding claims, wherein Laureth-9 is present in amounts of 0.1 to 10 wt.-% of the total weight of the composition, preferably in amounts of 0.2 to 8 wt.-% of the total weight of the composition, more preferred in amounts of 0.5 to 6 wt.-% of the total weight of the composition, even more preferred in amounts of 0.75 to 5 wt.-% of the total weight of the composition and most preferred in amounts of 1 to 4 wt.-% of the total weight of the composition.

10. The cosmetic composition of any of the preceeding claims in form of a cream, lotion, emulsion, solution, body milk, body spray, unguent, ointment, paste, detergent composition, shower gel, hair shampoo, conditioner or antiperspirant (deodorizing composition), or a decorative cosmetic product like make-up, rouge or tanning composition.

11. The cosmetic composition according to any of the preceeding claims for the care of irritated skin wherein the irritation is caused by dermatitis, neurodermitis, psoriasis, eczema, allergic reaction, heat, cold, sunburn and/or dryness.

12. The composition of any of the preceeding claims for topical use.

13. A combination of
a. Allantoin and Heliotropine, and
b. ionically bound and/or free thiocyanate ions or derivatives thereof for use in the care and/or treatment of dermatitis, neurodermitis, psoriasis, eczema, allergic reaction, heat, cold, sunburn and/or dryness.

14. Use of ionically bound and/or free thiocyanate ions or derivatives thereof in cosmetic compositions for improved effectiveness of incorporation and distribution of Allantoin and Heliotropine in said cosmetic compositions.

## Patentansprüche

1. Eine kosmetische Zusammensetzung, enthaltend:
a. Allantoin und Heliotropin und
b. ionisch gebundene und/oder freie Thiocyanationen oder Derivate davon.

2. Die kosmetische Zusammensetzung gemäß Anspruch 1, wobei die Thiocyanationen von Alkalimetallsalzen, Ammoniumsalzen oder Derivaten davon vorliegen, wobei bevorzugt die Alkalimetallsalze der Thiocyanate ausgewählt sind aus Natriumthiocyanat, Kaliumthiocyanat und/oder Ammoniumthiocyanat, weiter bevorzugt die Alkalimetallsalze der Thiocyanate ausgewählt sind aus Natriumthiocyanat und Kaliumthiocyanat.

3. Die kosmetische Zusammensetzung gemäß einem der vorhergehenden Ansprüche, wobei die Zusammensetzung außerdem Laureth-9 enthält.

4. Die kosmetische Zusammensetzung gemäß einem der vorhergehenden Ansprüche, wobei die Zusammensetzung außerdem einen oder mehrere Bestandteile einer kosmetischen Zusammensetzung, ausgewählt aus Öl(en), Wachs(en), Fettsäure(n), Detergenz(ien), Tensid(en), Emulgierungsmittel(n), insbesondere bevorzugt mindestens einen Glycerylester und/oder einen oder mehrere mehrwertige Alkohole enthält.

5. Die kosmetische Zusammensetzung gemäß einem der vorhergehenden Ansprüche, enthaltend Allantoin und ionisch gebundene und/oder freie Thiocyanationen oder Derivate davon in einem Gewichtsverhältnis von 10:1 bis 1:40, bevorzugt in einem Gewichtsverhältnis von 5:1 bis 1:20, weiter bevorzugt in einem Gewichtsverhältnis von 2:1 bis 1:10, weiter bevorzugt in einem Gewichtsverhältnis von 1:1 bis 1:8, weiter bevorzugt in einem Gewichtsverhältnis von 1:1 bis 1:6, weiter bevorzugt in einem Gewichtsverhältnis von 1:2 bis 1:5 und am meisten bevorzugt in einem Gewichtsverhältnis von 1:2 bis 1:4.

6. Die kosmetische Zusammensetzung gemäß einem der vorhergehenden Ansprüche, enthaltend Heliotropin und ionisch gebundene und/oder freie Thiocyanationen oder Derivate davon in einem Gewichtsverhältnis von 10:1 bis 1:40, bevorzugt in einem Gewichtsverhältnis von 5:1 bis 1:20, weiter bevorzugt in einem Gewichtsverhältnis von 2:1 bis 1:10 und am meisten bevorzugt in einem Gewichtsverhältnis von 1:1 bis 1:4.

7. Die kosmetische Zusammensetzung gemäß einem der vorhergehenden Ansprüche, wobei Allantoin und/oder Heliotropin unabhängig voneinander jeweils in Mengen von 0,01 bis 5 Gew.-% des Gesamtgewichts der Zusammensetzung vorliegt, bevorzugt in Mengen von 0,02 bis 3 Gew.-% des Gesamtgewichts der Zusammensetzung, weiter bevorzugt in Mengen von 0,05 bis 2 Gew.-% des Gesamtgewichts der Zusammensetzung, noch weiter bevorzugt in Mengen von 0,08 bis 1 Gew.-%, noch weiter bevorzugt in Mengen von 0,1 bis 0,8 Gew.-% und am meisten bevorzugt in Mengen von 0,2 bis 0,6 Gew.-% des Gesamtgewichts der Zusammensetzung.

8. Die kosmetische Zusammensetzung gemäß einem der vorhergehenden Ansprüche, wobei die ionisch gebundenen und/oder freien Thiocyanationen oder die Derivate davon in Mengen von 0,1 bis 1 Gew.-% des Gesamtgewichts der Zusammensetzung, bevorzugt in Mengen von 0,1 bis weniger als 1 Gew.-%, zum Beispiel in Mengen von 0,1 bis 0,98 Gew.-% des Gesamtgewichts der Zusammensetzung, bevorzugt in Mengen von 0,2 bis 0,95 Gew.-% des Gesamtgewichts der Zusammensetzung, weiter bevorzugt in Mengen von 0,3 bis 0,9 Gew.-% des Gesamtgewichts der Zusammensetzung, weiter bevorzugt in Mengen von 0,4 bis 0,85 Gew.-% des Gesamtgewichts der Zusammensetzung, weiter bevorzugt in Mengen von 0,5 bis 0,8 Gew.-% des Gesamtgewichts der Zusammensetzung, sogar weiter bevorzugt in Mengen von 0,6 bis 0,75 Gew.-% des Gesamtgewichts der Zusammensetzung und am meisten bevorzugt in Mengen von 0,65 bis 0,73 Gew.-% des Gesamtgewichts der Zusammensetzung vorliegen.

9. Die kosmetische Zusammensetzung gemäß einem der vorhergehenden Ansprüche, wobei Laureth-9 in Mengen von 0,1 bis 10 Gew.-% des Gesamtgewichts der Zusammensetzung, bevorzugt in Mengen von 0,2 bis 8 Gew.-% des Gesamtgewichts der Zusammensetzung, weiter bevorzugt in Mengen von 0,5 bis 6 Gew.-% des Gesamtgewichts der Zusammensetzung, noch weiter bevorzugt in Mengen von 0,75 bis 5 Gew.-% des Gesamtgewichts der Zusammensetzung und am meisten bevorzugt in Mengen von 1 bis 4 Gew.-% des Gesamtgewichts der Zusammensetzung vorliegt.

10. Die kosmetische Zusammensetzung gemäß einem der vorhergehenden Ansprüche in Form einer Creme, Lotion, Emulsion, Lösung, Körpermilch, eines Körpersprays, einer Salbe, Wundsalbe, Paste, Reinigungsmittelzusammensetzung, eines Duschgels, Haarshampoos, Conditioners oder Deos (deodorisierenden Zusammensetzungen) oder eines dekorativen Kosmetikprodukts, wie Make-up, Rouge oder einer Bräunungszusammensetzung.

11. Die kosmetische Zusammensetzung gemäß einem der vorhergehenden Ansprüche für die Pflege irritierter Haut, wobei die Irritation ausgelöst wird durch Dermatitis, Neurodermitis, Psoriasis, einem Ekzem, einer allergischen Reaktion, Hitze, Kälte, Sonnenbrand und/oder Trockenheit.

12. Die Zusammensetzung gemäß einem der vorhergehenden Ansprüche für eine topische Verwendung.

13. Eine Kombination von
a. Allantoin und Heliotropin, und
b. ionisch gebundenen und/oder freien Thiocyanationen oder Derivaten davon zur Verwendung bei der Pflege und/oder Behandlung von Dermatitis, Neurodermitis, Psoriasis, einem Ekzem, einer allergischen Reaktion, Wärme, Kälte, Sonnenbrand und/oder Trockenheit.

14. Verwendung von ionisch gebundenen und/oder freien Thiocyanationen oder Derivaten davon in kosmetischen Zusammensetzungen zur verbesserten Wirksamkeit der Einbringung und Verteilung von Allantoin und Heliotropin in diesen kosmetischen Zusammensetzungen.

## Revendications

1. Composition cosmétique comprenant :
a) de l'allantoïne et de l'héliotropine,
b) et des ions thiocyanate ou des dérivés de ces ions, libres et/ou liés par liaison ionique.

2. Composition cosmétique conforme à la revendication 1, dans laquelle les ions thiocyanate se présentent sous forme de sels de métal alcalin, de sels d'ammonium ou de dérivés de tels sels, étant entendu que les sels thiocyanates de métal alcalin sont de préférence choisis parmi du thiocyanate de sodium, du thiocyanate de potassium et/ou du thiocyanate d'ammonium, et que, mieux encore, les sels thiocyanates de métal alcalin sont choisis parmi du thiocyanate de sodium et du thiocyanate de potassium.

3. Composition cosmétique conforme à l'une des revendications précédentes, laquelle composition comprend en outre du laureth-9.

4. Composition cosmétique conforme à l'une des revendications précédentes, laquelle composition comprend en outre un ou plusieurs autre(s) ingrédient(s) de composition cosmétique, choisi(s) parmi les huile(s), cire(s), acide(s) gras, détergent(s), tensioactif(s) et émulsifiant(s), avec une préférence particulière pour au moins un ester de glycéryle et/ou un ou plusieurs polyol(s).

5. Composition cosmétique conforme à l'une des revendications précédentes, qui comprend de l'allantoïne et des ions thiocyanate ou des dérivés de ces ions, libres et/ou liés par liaison ionique, en un rapport pondéral de 10/1 à 1/40, de préférence en un rapport pondéral de 5/1 à 1/20, mieux encore en un rapport pondéral de 2/1 à 1/10, mieux encore en un rapport pondéral de 1/1 à 1/8, mieux encore en un rapport pondéral de 1/1 à 1/6, mieux encore en un rapport pondéral de 1/2 à 1/5, et surtout en un rapport pondéral de 1/2 à 1/4.

6. Composition cosmétique conforme à l'une des revendications précédentes, qui comprend de l'héliotropine et des ions thiocyanate ou des dérivés de ces ions, libres et/ou liés par liaison ionique, en un rapport pondéral de 10/1 à 1/40, de préférence en un rapport pondéral de 5/1 à 1/20, mieux encore en un rapport pondéral de 2/1 à 1/10, et surtout en un rapport pondéral de 1/1 à 1/4.

7. Composition cosmétique conforme à l'une des revendications précédentes, dans laquelle l'allantoïne et/ou l'héliotropine est ou sont présente(s), indépendamment l'une de l'autre, en des proportions de 0,01 à 5 %, en poids rapporté au poids total de la composition, de préférence en des proportions de 0,02 à 3 %, en poids rapporté au poids total de la composition, mieux encore en des proportions de 0,05 à 2 %, en poids rapporté au poids total de la composition, mieux encore en des proportions de 0,08 à 1 %, en poids rapporté au poids total de la composition, mieux encore en des proportions de 0,1 à 0,8 %, en poids rapporté au poids total de la composition, et surtout, en des proportions de 0,2 à 0,6 %, en poids rapporté au poids total de la composition.

8. Composition cosmétique conforme à l'une des revendications précédentes, dans laquelle les ions thiocyanate ou les dérivés de ces ions, libres et/ou liés par liaison ionique, sont présents en des proportions de 0,1 à 1 %, en poids rapporté au poids total de la composition, et de préférence en des proportions de 0,1 à moins de 1 % en poids, par exemple en des proportions de 0,1 à 0,98 %, en poids rapporté au poids total de la composition, de préférence en des proportions de 0,2 à 0,95 %, en poids rapporté au poids total de la composition, mieux encore en des proportions de 0,3 à 0,9 %, en poids rapporté au poids total de la composition, mieux encore en des proportions de 0,4 à 0,85 %, en poids rapporté au poids total de la composition, mieux encore en des proportions de 0,5 à 0,8 %, en poids rapporté au poids total de la composition, mieux encore en des proportions de 0,6 à 0,75 %, en poids rapporté au poids total de la composition, et surtout, en des proportions de 0,65 à 0,73 %, en poids rapporté au poids total de la composition.

9. Composition cosmétique conforme à l'une des revendications précédentes, dans laquelle le laureth-9 est présent en une proportion de 0,1 à 10 %, en poids rapporté au poids total de la composition, de préférence en une proportion de 0,2 à 8 %, en poids rapporté au poids total de la composition, mieux encore en une proportion de 0,5 à 6 %, en poids rapporté au poids total de la composition, mieux encore en une proportion de 0,75 à 5 %, en poids rapporté au poids total de la composition, et surtout, en une proportion de 1 à 4 %, en poids rapporté au poids total de la composition.

10. Composition cosmétique conforme à l'une des revendications précédentes, sous forme de crème, de lotion, d'émulsion, de solution, de lait corporel, de spray corporel, d'onguent, de pommade, de pâte, de composition détergente, de gel-douche, de shampooing, de crème traitante ou de produit anti-transpiration (composition de déodorant), ou de produit cosmétique de beauté tel que produit de maquillage, rouge à joues, ou composition bronzante.

11. Composition cosmétique conforme à l'une des revendications précédentes pour soins d'une peau irritée, quand l'irritation est causée par une dermatite, une névrodermite, un psoriasis, un eczéma, une réaction allergique, un coup de chaleur, un coup de froid, un coup de soleil et/ou un état de sécheresse.

12. Composition conforme à l'une des revendications précédentes pour utilisation topique.

13. Combinaison
a) d'allantoïne et d'héliotropine,
b) et d'ions thiocyanate ou de dérivés de ces ions, libres et/ou liés par liaison ionique,
pour utilisation dans les soins et/ou le traitement d'une dermatite, d'une névrodermite, d'un psoriasis, d'un eczéma, d'une réaction allergique, d'un coup de chaleur, d'un coup de froid, d'un coup de soleil et/ou d'un état de sécheresse.

14. Utilisation d'ions thiocyanate ou de dérivés de ces ions, libres et/ou liés par liaison ionique, dans des compositions cosmétiques, pour augmenter l'efficacité de l'allantoïne et de l'héliotropine en améliorant leur incorporation dans lesdites compositions cosmétiques et leur répartition au sein de celles-ci.
